# EUROPÄISCHE PATENTSCHRIFT

(11) **EP 2 945 904 B1**
(45) Veröffentlichungstag und Bekanntmachung des Hinweises auf die Patenterteilung: **04.01.2017**
(21) Anmeldenummer: 14701497.1
(22) Anmeldetag: 15.01.2014
(51) Int. Cl.: C01B 3/36, C07C 2/78, C07C 11/24

(54) **VERFAHREN ZUR HERSTELLUNG VON ACETYLEN UND SYNTHESEGAS**
METHOD FOR PRODUCING ACETYLENES AND SYNGAS
PROCÉDÉ DE PRODUCTION D'ACÉTYLÈNE ET DE GAZ DE SYNTHÈSE

(30) Priorität: 16.01.2013 US 201361753019 P
(43) Veröffentlichungstag der Anmeldung: 25.11.2015
(73) Patentinhaber: BASF SE, 67056 Ludwigshafen am Rhein (DE)
(72) Erfinder: VICARI, Maximilian, 67117 Limburgerhof (DE); WEICHERT, Christian, 67098 Bad Dürkheim (DE); GROßSCHMIDT, Dirk, 68259 Mannheim (DE); RUSS, Michael, 67354 Römerberg (DE); EHRHARDT, Kai Rainer, 67346 Speyer (DE); NEUHAUSER, Horst, 67373 Dudenhofen (DE); HAYES, Michael L., Gonzales, Louisiana 70737 (US)
(86) Internationale Anmeldenummer: PCT/EP2014/050652
(87) Internationale Veröffentlichungsnummer: WO 2014/111396

(56) Entgegenhaltungen:
- WO-A1-2007/096271
- WO-A1-2012/062784
- US-A- 3 843 744
- US-A- 5 824 834
- Pässler, P et al.: "Acetylene, 4. Production, 4.2 Partial Combustion Processes" In: "Ullmann's Encyclopedia of Industrial Chemistry", 15. Oktober 2011 (2011-10-15), Wiley-VCH Verlag, internet, XP002722234, Seite 284-293, DOI: 10.1002/14356007.a01_097.pub4, Seite 287 - Seite 289

## Beschreibung

Die vorliegende Erfindung betrifft ein Verfahren zur Herstellung von Acetylen und Synthesegas durch partielle Oxidation von Kohlenwasserstoffen mit Sauerstoff.

Die obige partielle Oxidation ist eine Hochtemperaturreaktion, die üblicherweise in einem Reaktorsystem, umfassend eine Mischeinrichtung, einen Brennerblock sowie eine Quencheinrichtung, durchgeführt wird, und beispielsweise in Ullmanns Encyclopedia of Industrial Chemistry (5th Edition, Volume A1, Seiten 97 - 144) beschrieben ist.

Gemäß Ullmanns Encyclopedia of Industrial Chemistry (5th Edition, Volume A1 Seiten 97 - 144) erfolgt die Aufheizung der Einsatzstoffe getrennt in Vorheizern. Die aufgeheizten Einsatzstoffe werden in einer Mischeinrichtung gemischt und über einen Mischdiffusor einem Brenner und weiter einem Feuerraum zugeführt. Stromab des Feuerraums wird mittels Düsen ein wässriges Quenchmedium dem Spaltgas zugeführt und dieses schnell auf etwa 80 -90 °C abgekühlt. Der Prozess wird durch geeignete Wahl der Sauerstoffzahl λ so betrieben (λ < 0,31), dass die Ausbeute an Acetylen bezogen auf das trockene Spaltgas optimal groß wird (> 8 %). Hierbei wird unter Sauerstoffzahl λ das Verhältnis aus der tatsächlich vorhandenen Sauerstoffmenge zur stöchiometrisch notwendigen Sauerstoffmenge verstanden, die für die vollständige Verbrennung der Einsatzstoffe erforderlich ist. Dabei wird die Rußbeladung des Spaltgases maximal. Der im Feuerraum aus der Gasphase gebildete Ruß wird zum Teil durch den Quench, in einer anschließenden Kühlkolonne und einem daran anschließenden Elektrofilter abgeschieden. Der wertprodukthaltige Produktgasstrom wird getrennt über die Kühlkolonne abgeführt. Nach dem Elektrofilter ist die Rußkonzentration im restlichen Spaltgas (ohne Wertprodukte) auf etwa 1 mg/m³ gesunken. Der im Prozesswasser aus dem Quench, der Kühlkolonne und dem Elektrofilter enthaltene Ruß besitzt einen hohen Kohlenwasserstoffanteil und ist daher hydrophob, was ihn auf dem Prozesswasser aufschwimmen lässt. Daher wird dieses rußbeladene Prozesswasser über sogenannte offene Rußrinnen mit Oberflächen-Partikel-Abscheidern geleitet. Die aufschwimmenden Rußanteile werden dabei abgetrennt und einer Feuerung zugeführt. Das so gereinigte Prozesswasser wird anschließend über einen offenen Kühlturm gefahren und darin abgekühlt. Dabei und während der Fest-Flüssig-Trennung zuvor wird ein Großteil der flüssig und gasförmig im Prozesswasser gebundenen Kohlenwasserstoffe, insbesondere Aromate, Alkine, Benzol-Toluol-Xylol, etc. zusammen mit Teilen des Prozesswassers in die Umgebungsluft emittiert. Anschließend wird der so entstandene Verlust an Prozesswasser durch Zugabe kompensiert und der Wasserkreislauf Richtung Kühlkolonne und Quench geschlossen.

Die Emissionen an Kohlenwasserstoffen aus dem Prozesswasser aus dem Kühlturm (d. h. bei einer offenen Prozesswasserfahrweise) sind jedoch unter den geltenden Umweltschutzauflagen nicht mehr tragbar. Auch eine geschlossene Prozesswasserfahrweise ist keine tragbare Lösung, da sich hierbei die Kohlenwasserstoffe anreichern und zu Polymerisation und Verstopfung der Anlage führen würden.

Eine weitere Emissionsquelle stellen die offenen Rußrinnen dar. Die aus dem Prozesswasser in den Rußrinnen abgeschiedenen Feststoffe müssen vor einer möglichen kommerziellen Vermarktung aufwändig getrocknet werden, was dieselbe unattraktiv macht.

Ein weiteres Verfahren zur Herstellung von Acetylen und Synthesegas durch partielle Oxidation von Kohlenwasserstoffen mit Sauerstoff ist in US 5,824,834 beschrieben. Dabei handelt es sich um ein Rußmengen-optimiertes, geschlossenes Wasserquenchverfahren, das mit einem mageren Feedstrom betrieben wird, und zwar mit einem Feedstrom mit einer Sauerstoffzahl λ > 0,31. Das Verfahren hat jedoch den Nachteil einer reduzierten Ausbeute an Wertprodukt Acetylen.

Bei dieser Verfahrensvariante wird das wässrige Quenchmedium ebenfalls mittels Düsen dem Spaltgas zugeführt und dieses schnell auf etwa 80 -90 °C abgekühlt. Der im Feuerraum aus der Gasphase gebildete Ruß wird zum Teil durch den Quench, eine anschließende mit rezirkulierendem Wasser betriebene Kühlkolonne und einem daran anschließenden Elektrofilter abgeschieden. Der werthaltige Produktgasstrom wird getrennt über die Kühlkolonne abgeführt. Der Prozess wird hierbei durch Wahl der Sauerstoffzahl λ so betrieben (λ > 0,31), dass die anfallende Rußmenge im Spaltgas so gering ist, dass allein durch die Ausschleusung des anfallenden Reaktionswassers aus der Verbrennung der stationäre Betrieb gewährleistet werden kann. Dadurch reduziert sich jedoch der Acetylengehalt im trockenen Spaltgas um 2 Prozentpunkte gegenüber dem vorstehend beschriebenen Verfahren auf etwa 6 Vol.-%. Damit wird eine geschlossene, also gegenüber der Umwelt abgetrennte Wasserquench-Fahrweise ermöglicht. Der Vorteil gegenüber der zuvor beschriebenen Verfahrensvariante ist somit die Möglichkeit des geschlossenen Betriebes ohne weitere Trennapparaturen. Der Nachteil sind Ausbeuteeinbußen bezüglich des Wert- und Zielprodukts Acetylen. Zudem gilt ebenfalls, dass die aus dem Prozesswasser abgeschiedenen Feststoffe vor einer möglichen kommerziellen Vermarktung aufwändig getrocknet werden müssen, was dieselbe unattraktiv macht.

Ein drittes Verfahren zur Herstellung von Acetylen und Synthesegas durch partielle Oxidation von Kohlenwasserstoffen mit Sauerstoff ist in EP-A 12171956 beschrieben. Dabei handelt es sich um ein Verfahren, das die Vorteile der beiden vorstehend beschriebenen Verfahren, das heißt optimierte Ausbeute an Wertprodukt Acetylen entsprechend Ullmanns Encyclopedia of Industrial Chemistry, 5th Edition, Volume A1, Seiten 97 - 144 und Einhaltung geltender Umweltschutzauflagen entsprechend US 5,824,834 kombiniert, und als Ziel formuliert, Nachteile, das heißt nicht zeitgemäße Missachtung von Umweltschutzauflagen des erstgenannten obigen Verfahrens sowie deutliche Ausbeuteeinbußen des zweitgenannten Verfahrens zu minimieren. Es sei hierbei darauf hingewiesen, dass gemäß Ullmanns Encyclopedia of Industrial Chemistry (5th Edition, Volume A1, Seiten 97 - 144) sowohl die Menge an anfallendem Ruß, Koks und Teer sowie an höheren Alkinen und Naphthalin bei Fahrweisen mit Sauerstoffzahlen von λ < 0,31 überproportional ansteigen und nicht mehr in hinreichendem Maße durch die in US 5,824,834 beschriebenen Abscheidekonzepte abzutrennen und rückzuhalten sind, um die geltenden Umweltschutzauflagen zu erfüllen.

Ein weiteres Verfahren zur Herstellung von Acetylen und Synthesegas durch partielle Oxidation von Kohlenwasserstoffen mit Sauerstoff ist in EP-A 1 989 160 beschrieben. Dabei handelt es sich um eine Erweiterung des Verfahrens der US 5,824,834 dahingehend, dass die restlichen sehr feinen Feststoffanteile (Ruß, Teer, Koks) im Produktgas mittels Produktgaskompressoren abgeschieden werden. Hierbei scheiden sich die im bereits vorgereinigten Produktgas angefallenen Feststoffe im direkt in den Produktgaskompressor eingespritzten Kühlwasser ab und werden aus diesem ausgetragen. Nachteilig ist jedoch, dass der anfallende Feststoff hierbei im Wasser gebunden ausgetragen wird. Dies erfordert zur kommerziellen (trockenen) Vermarktung dieser Feststoffe eine aufwändige und kostenintensive Nachbehandlung in Form einer Trocknung, was in der Regel aus Kostengründen einer kommerziell attraktiven Vermarktung im Wege steht.

Daher wird im Zuge der europäischen Patentanmeldung mit der Anmeldenummer EP 12171956.1 im Wesentlichen die Kombination der drei Verfahrenskonzepte aus Ullmanns Encyclopedia of Industrial Chemistry (5th Edition, Volume A1, Seiten 97-144), US 5,824,834 und EP-A 1 989 160 beschrieben. Zudem wird die Abscheidung von flüssigen und gasförmigen unerwünschten Nebenprodukten (im Wesentlichen höhere Alkine und Naphthalin) mittels Teilverdampfung (Flash) beschrieben.

Der obige Stand der Technik gibt jedoch keinen Hinweis auf das Problem des erhöhten Anfalls von Ruß, Koks und Teer und wie diese trocken abgeschieden werden können. Es war demgegenüber Aufgabe der Erfindung, ein Verfahren zur Herstellung von Acetylen und Synthesegas durch partielle Oxidation von Kohlenwasserstoffen zur Verfügung zu stellen, das die Vorteile der obigen Verfahren kombiniert, das heißt sowohl eine hohe Ausbeute an Wertprodukt Acetylen sowie die Einhaltung der geltenden Umweltschutzauflagen durch hinreichende Abscheidung und Rückhaltung von unerwünschten gasförmigen und/oder flüssigen Nebenprodukten gewährleistet und das darüber hinaus die hinreichende trockene Abtrennung und Rückhaltung von festen unerwünschten Nebenprodukten (Teer, Koks, Ruß) ermöglicht.

Die Aufgabe wird gelöst durch ein kontinuierliches Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter Sauerstoff, enthaltender Einsatzstrom
- getrennt voneinander, in Vorheizern, aufgeheizt, in einem Verhältnis der Massenströme des zweiten Einsatzstromes zum ersten Einsatzstrom entsprechend einer Sauerstoffzahl von kleiner oder gleich 0,31 in einer Mischeinrichtung gemischt werden, wobei man unter Sauerstoffzahl das Verhältnis aus der tatsächlich im zweiten Einsatzstrom vorhandenen Sauerstoffmenge zur stöchiometrisch notwendigen Sauerstoffmenge versteht, die für die vollständige Verbrennung des einen oder der mehreren, im ersten Einsatzstrom enthaltenen Kohlenwasserstoffe erforderlich ist,
- über einen Brennerblock einem Feuerraum zugeführt werden, worin die partielle Oxidation der Kohlenwasserstoffe stattfindet,
- unter Erhalt eines ersten Spaltgasstromes I_{g},
dadurch gekennzeichnet, dass
- der erste Spaltgasstrom I_{g} in einem Vorquench durch Eindüsen eines wässrigen Quenchmediums auf eine Temperatur im Bereich von 100 bis 1.000 °C, unter Erhalt eines zweiten Spaltgasstromes II_{g} abgekühlt wird,
- aus dem zweiten Spaltgasstromes II_{g} in einem Feststoff-Gas-Trennapparat 50 bis 90 % der darin enthaltenen Feststoffe abgetrennt werden, unter Erhalt eines Feststoffstromes I_{f} sowie eines dritten Spaltgasstromes III_{g},
- der dritte Spaltgasstrom III_{g} in einem Totalquench durch Eindüsen von Wasser auf 80 bis 90 °C abgekühlt wird, unter Erhalt eines vierten Spaltgasstromes IV_{g} sowie eines ersten Prozesswasserstromes I_{liq},
- der vierte Spaltgasstrom IV_{g} in einem oder mehreren Waschapparaten einer Feinabtrennung von Feststoff unterworfen wird, unter Erhalt eines oder mehrerer Prozesswasserströme II_{liq}, III_{liq} sowie eines Produktgasstromes VI_{g},
- die Prozesswasserströme I_{liq}, II_{liq}, III_{liq} zu einem vereinigten Prozesswasserstrom IV_{liq} zusammengeführt werden,
- der vereinigte Prozesswasserstrom IV_{liq} teilweise, als Strom V_{liq}, in den Totalquench zurückgeführt und im Übrigen, als Strom VI_{liq}, einer Reinigung durch Teilverdampfung in einem einstufigen Entspannungsbehälter unterworfen wird, wobei der Strom VI_{liq} zu einem Anteil von 0,01 bis 10 Gew.-%, bezogen auf das Gesamtgewicht desselben, verdampft wird, unter Erhalt eines gereinigten Prozesswasserstromes VII_{liq}, der über eine Rückkühleinrichtung abgekühlt, teilweise, als Strom VIII_{liq} in einen oder mehrere des einen oder der mehreren Waschapparate rezykliert, und im Übrigen, als Strom IX_{liq}, ausgeschleust und dem behandlungsbedürftigen Abwasser zugeführt wird.

Es wurde gefunden, dass durch die erfindungsgemäße Verfahrensführung, durch Vorquenchen des Spaltsgasstromes aus einem Verfahren zur kontinuierlichen Herstellung von Acetylen und Synthesegas auf eine Temperatur im Bereich von 100 bis 1.000 °C sowohl ein unerwünschtes Auskondensieren von Reaktionswasser, Quenchwasser oder Teeren vor der Zuführung des hierbei erhaltenen Spaltgasstromes in einem nachfolgenden Feststoff-Gas-Trennapparat, als auch eine thermische Überlastung des Feststoff-Gas-Trennapparats vermieden werden und zudem ein Abbruch der Synthese-Reaktion und damit eine optimale Acetylen-Ausbeute gewährleistet werden kann.

Indem der Feststoff-Gas-Trennapparat erfindungsgemäß dergestalt betrieben wird, dass darin 50 bis 90 % der Feststoffe aus dem im Vorquench erhaltenen Spaltgasstrom abgetrennt werden, ist die im Spaltgas verbleibende Rußmenge so gering, dass allein durch die Ausschleusung eines Teilstromes des gereinigten Prozesswasserstromes, insbesondere in einem Mengenstrom entsprechend dem aus bei der partiellen Oxidation von Kohlenwasserstoffen mit Sauerstoff anfallenden Reaktionswasser in das behandlungsbedürftige Abwasser ein stationärer, kontinuierlicher Betrieb der Anlage gewährleistet werden kann.

Es wurde überraschend gefunden, dass durch einen weiteren dem Vorquench nachgeschalteten einfachen Feststoff-Gas-Trennapparat die gesamte Feststoffabscheiderate in das Prozesswasser hinreichend hoch ist und somit eine zusätzliche Abtrennung in einem aufwändig und mit großem Energiebedarf betriebenen Feststoff-Gas-Trennapparat in Form eines Elektrofilters überflüssig macht. Zudem ist durch das vorgeschlagene Konzept der Feststoff-Abscheidung der Feststoffgehalt im erhaltenen Prozesswasser so gering, dass durch kontinuierliche Ausschleusung von Prozesswasser, insbesondere in einer Menge entsprechend dem in der partiellen Oxidation von Kohlenwasserstoffen mit Sauerstoff anfallendem Reaktionswasser in das behandlungsbedürftige Abwasser ein kontinuierlicher Betrieb des Verfahrens ohne weitere, aufwändige und wasserintensive Fest-Flüssig-Abtrenneinrichtung (Rußrinnen) möglich ist.

Das erfindungsgemäße Verfahren ist unabhängig von der konkreten Ausbildung des eingesetzten Reaktorsystems, umfassend Mischeinrichtung, Brennerblock und Quencheinrichtung.

Im Folgenden werden die bevorzugten, üblicherweise eingesetzten Reaktorsysteme näher erläutert:
Die Ausgangsstoffe, das heißt ein Kohlenwasserstoffe enthaltender erster Einsatzstrom, insbesondere Erdgas, und ein zweiter, Sauerstoff enthaltender Gasstrom, werden getrennt voneinander aufgeheizt, üblicherweise bis hin zu 600 °C. In einer Mischeinrichtung werden die Reaktanden intensiv vermischt und nach Durchströmen eines Brennerblocks in einem Feuerraum zur exothermen Reaktion gebracht. Der Brennerblock besteht üblicherweise aus einer Vielzahl von parallelen Kanälen, in denen die Strömungsgeschwindigkeit der zündfähigen Sauerstoff-Kohlenwasserstoff-Mischung höher ist als die Flammengeschwindigkeit, um ein Durchschlagen der Flamme in die Mischeinrichtung zu verhindern. Der metallische Brennerblock wird gekühlt, um den thermischen Belastungen standzuhalten. Je nach Aufenthaltszeit in der Mischeinrichtung besteht die Gefahr der Vor- und Rückzündung aufgrund der begrenzten thermischen Stabilität der Mischungen. Hierzu wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden in der Mischeinrichtung. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch die zylinderförmige Geometrie des Feuerraums aus. Der Brennerblock weist vorzugsweise hexagonal angeordnete Durchführungsbohrungen auf. In einer Ausführungsform sind z. B. 127 Bohrungen ä 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in dem die Flamme der Acetylen und Synthesegas bildenden partiellen Oxidationsreaktion stabilisiert wird, ist üblicherweise ebenfalls von zylindrischem Querschnitt, ist wassergekühlt und entspricht im Erscheinungsbild dem eines kurzen Rohres, von z.B. 180 bis 533 mm Durchmesser und 380 bis 450 mm Länge. In Höhe des Brennerblocks wird sowohl in axialer als auch in radialer Richtung sogenannter Hilfssauerstoff dem Feuerraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt.

Der gesamte Brenner aus Brennerblock und Feuerraum wird bevorzugt in einen Quenchbehälter größeren Querschnitts über einen Flansch von oben eingehängt. Auf Höhe der Austrittsebene aus dem Feuerraum sind am äußeren Umfang desselben Quenchdüsen auf einem oder mehreren Quenchverteilerringen installiert, die das Quenchmedium mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe, das Reaktionsgemisch extrem schnell abzukühlen, so dass Folgereaktionen, das heißt insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Bei dem vorliegenden technischen Verfahren entsteht neben Acetylen im Wesentlichen Wasserstoff, Kohlenmonoxid und Ruß. Die in der Flammenfront gebildeten Rußpartikel können als Keime an den Feuerraum-Seitenwänden anhaften, worauf es bei geeigneten physikalisch-chemischen Bedingungen zum Aufwachsen, Ablagern und Anbacken von Koksschichten kommt.

Diese Ablagerungen werden periodisch im Bereich der Feuerraumwände mittels einer Stocherreinrichtung mechanisch abgereinigt, wie beispielsweise in Ullmanns Encyclopedia of Industrial Chemistry 5th Edition, Volume A1, Seiten 97 - 144 oder in US 5,824,834 beschrieben.

Zu einer weiteren bevorzugten Ausführungsform weist das Reaktorsystem, eine gespülte Reaktorseitenwand auf, wie in WO-A 2012/062784 beschrieben, oder eine gespülte Reaktorstirnfläche, wie in WO-A 2012/062584 beschrieben. Bei diesen Ausführungsformen kann das zuvor beschriebene mechanische Stochern entfallen und damit wird vermieden, das großskalige Feststoffteile stromab weitergetragen werden und dort den weiteren Prozess behindern - z.B. durch Verstopfen von Trenn- und/oder Wärmetauscherapparaten.

In einer vorteilhaften Ausführungsform werden aus dem zweiten Spaltgasstrom II_{g} im Feststoff-Gas-Trennapparat 60 bis 80 % der darin enthaltenen Feststoffe abgetrennt.

Der im Feststoff-Gas-Trennapparat abgeschiedene Feststoffstrom I_{f} enthält überwiegend Teere, Ruße und Kokse.

Der Feststoff-Gas-Trennapparat ist vorteilhaft ein Zyklonabscheider.

Vorteilhaft wird der erste Spaltgasstrom I_{g} im Vorquench auf eine Temperatur im Bereich von 200 bis 650 °C abgekühlt.

Weiter vorteilhaft wird der erste Spaltgasstrom I_{g} im Vorquench auf eine Temperatur im Bereich von 250 bis 400 °C abgekühlt.

Der Vorquench ist vorteilhaft ein Wasserquench.

Energetisch besonders vorteilhaft ist eine Ausführungsform, wonach der Vorquench einen Wärmetauscher zur Auskopplung von Reaktionswärme in Form von Hochdruckdampf umfasst.

Bevorzugt wird als erster Waschapparat ein Venturiwäscher und als zweiter Waschapparat eine Quenchkolonne eingesetzt.

Die Rückführung des gereinigten Prozesswasserteilstromes VIII_{liq} erfolgt bevorzugt in dem zweiten Waschapparat.

In einer bevorzugten Ausführungsform ist die Rückkühleinrichtung eine geschlossene Rückkühleinrichtung.

In einer weiteren bevorzugten Ausführungsform ist die Rückkühleinrichtung ein offener Kühlturm. In dieser Verfahrensvariante wird bevorzugt der gesamte gereinigte Prozesswasserstrom in das Verfahren rezykliert.

Die Erfindung wird im Folgenden anhand einer Zeichnung sowie von Ausführungsbeispielen näher erläutert.

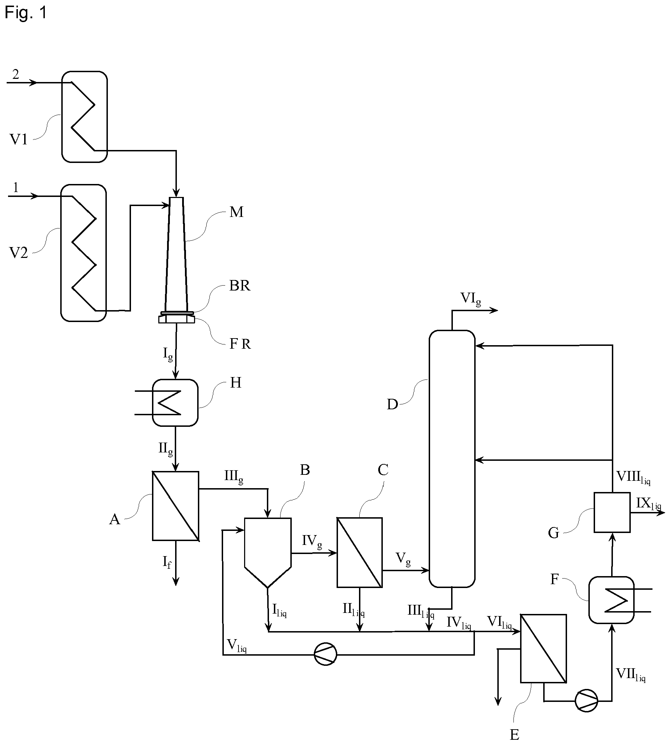

Figur 1 zeigt die schematische Darstellung einer bevorzugten erfindungsgemäßen Anlageausführung.

Der in Figur 1 dargestellten bevorzugten Anlage zur Durchführung des erfindungsgemäßen Verfahrens wird ein erster Einsatzstrom 1, enthaltend einen oder mehrere Kohlenwasserstoffe sowie ein zweiter Sauerstoff enthaltender Einsatzsstrom 2 zugeführt, über Vorzeizer V1 bzw. V2 voneinander getrennt vorgeheizt, in einer Mischeinrichtung M gemischt, über einen Brennerblock BR einem Feuerraum FR zugeführt unter Erhalt eines ersten Spaltgasstromes I_{g}, der einem Vorquench H zugeführt und darin durch Eindüsen eines wässrigen, in der Figur nicht dargestellten Quenchmediums auf 100 bis 1.000 °C gequencht wird. Dabei wird in der in der Figur dargestellten bevorzugten Ausführungsform Reaktionswärme aus dem Vorquench H in Form von Hochdruckdampf ausgekoppelt. Der aus dem Vorquench H austretende zweite Spaltgasstrom II_{g} wird einem Feststoff-Gas-Trennapparat A zugeführt, der so ausgelegt ist, dass darin 50 bis 90 % der im zweiten Spaltgasstrom II_{g} enthaltenen Feststoffe, insbesondere Ruße, Teere und Kokse, abgetrennt werden, unter Erhalt eines Feststoffstromes I_{f}, der abgezogen wird sowie eines dritten Spaltgasstromes III_{g}, der einem Totalquench B zugeführt und darin durch direkte Wassereindüsung auf 80 bis 90 °C abgekühlt wird, unter Erhalt eines vierten Spaltgasstromes IV_{g} sowie eines ersten Prozesswasserstromes I_{liq}. Der vierte Spaltgasstromes IV_{g} wird in der in der Figur dargestellten bevorzugten Ausführungsform, einem ersten Waschapparat C zugeführt, und darin in einen fünften Spaltgasstrom V_{g} sowie einen zweiten Prozesswasserstrom II_{liq} aufgetrennt. Der Spaltgasstrom V_{g} wird einem zweiten Waschapparat D zugeführt und darin in einen Produktgasstrom VI_{g} sowie einen weiteren Prozesswasserstrom III_{liq} aufgetrennt. Die Prozesswasserströme I_{liq}, II_{liq}, III_{liq} werden zu einem vereinigten Prozesswasserstrom IV_{liq} zusammengeführt, der teilweise als Prozesswasserstrom V_{liq} in den Totalquench B zurückgeführt und im Übrigen als Prozesswasserstrom VI_{liq} einem einstufigen Entspannungsbehälter E zugeführt wird, worin eine Teilverdampfung erfolgt, unter Erhalt eines gereinigten Prozesswasserstromes VII_{liq}, der über eine Rückkühleinrichtung F abgekühlt und über ein Ventil G teilweise als Prozesswasserstrom IX_{liq} ausgeschleust und im Übrigen, als Prozesswasserstrom VIII_{liq}, in der Figur dargestellten bevorzugten Ausführungsform in zwei Teilströmen, in den zweiten Waschapparat D rezykliert wird. Bevorzugt wird der Massenstrom des Prozesswasserstromes IX_{liq} dergestalt eingestellt, dass die Menge an Wasser ausgeschleust wird, die bei der Reaktion, das heißt der partiellen Oxidation von Kohlenwasserstoffen mit Sauerstoff, anfällt.

Der Produktgasstrom VI_{g} wird als Wertproduktstrom, enthaltend im Wesentlichen Acetylen, Kohlenmonoxid und Wasserstoff, als Rohproduktgas einer Feinreinigung und Produktgastrennung zugeführt und in die entsprechende chemische Wertschöpfungskette eingespeist.

### Ausführungsbeispiele

### Vergleichsbeispiel:

Ohne Prozesswasserreinigung der gasförmigen und flüssigen unerwünschten Nebenprodukte fallen in einer Anlage entsprechend der schematischen Darstellung in Ullmanns Encyclopedia of Industrial Chemistry 5th Edition, Volume A1, Seiten 97 - 144 spezifisch für 1 t Acetylen bei einer Durchführung der partiellen Oxidation mit einer Sauerstoffzahl von 0,29 die folgenden Emissionen aus den offenen Rußrinnen und der Abluft des Kühlturms an:

| | Rußrinnen [kg] | Kühlturm [kg] | gesamt [kg] |
|---|---|---|---|
| CO | 0.303 | 0.363 | 0.667 |
| Methan | 5.67E-02 | 8.46E-02 | 0.141 |
| Ethan | 7.63E-03 | 1.21E-02 | 0.020 |
| Ethylen | 6.80E-03 | 2.88E-02 | 0.036 |
| Acetylen | 1.57E-01 | 6.05E+00 | 6.203 |
| Propen | 5.16E-04 | 1.88E-03 | 0.002 |
| Propadien | 9.83E-04 | 3.58E-03 | 0.005 |
| Propin | 2.29E-03 | 1.01E-01 | 0.103 |
| Butenin | 1.65E-03 | 4.58E-02 | 0.047 |
| Butadiin | 7.39E-03 | 8.91E-01 | 0.898 |
| Benzol | 2.29E-03 | 1.60E-01 | 0.162 |
| Naphthalin | 5.14E-04 | 1.40E-02 | 0.014 |

Darüber hinaus werden 57 kg/t Acetylen aus dem Prozesswasser über Rußrinnen und Elektrofilter nass abgeschieden.

### Ausführungsbeispiel nach der Erfindung:

Bei der Durchführung einer partiellen Oxidation von Erdgas mit Sauerstoff, bei einem Verhältnis der Massenströme des Erdgasstromes zum Sauerstoff enthaltenden Gasstrom entsprechend einer Sauerstoffzahl von 0,29 und entsprechend unter Erhalt einer hohen Ausbeute, von größer als 8 %, an Wertprodukt Acetylen wurde als Vorquench H ein Heißzyklonabscheider, als erster Waschapparat C ein Venturiwäscher und als zweiter Waschapparat D eine Kühlkolonne betrachtet.

Im Verfahren entsteht 65,57 kg Ruß pro Tonne Acetylen.

Bei einem Abscheidegrad von 80 % des Rußes im Vorquench H, das heißt einer trockenen Abscheidung, verbleibt nach dem Vorquench H ein Restgehalt von 13,11 kg Ruß pro Tonne Acetylen im Spaltgas.

Bei einem Abscheidegrad von 90 % über eine nasse Feinabscheidung im Venturiwäscher C verbleiben im Spaltgasstrom V_{g}, dass der den Venturiwäscher C verlässt, 1,31 kg Ruß pro Tonne Acetylen. Bei der weiteren nassen Feinabscheidung in der Kühlkolonne D, mit einem Abscheidegrad von 30 %, verbleibt im Spaltgasstrom VI_{g} ein Restgehalt von 0,92 kg Ruß pro Tonne Acetylen, der tolerierbar ist.

Im obigen Verfahren wird somit 80 % des gesamten Feststoffanteiles aus dem Spaltgas über einen Heißgaszyklon als Vorquench H trocken abgeschieden, so dass eine Vermarktung desselben ohne weitere energieaufwändige Trocknungsschritte möglich ist. Demgegenüber werden nur noch die restlichen 20 % des Feststoffanteiles nass über einfache, hoch effiziente Trennapparate, das heißt Venturiwäscher und Kühlkolonne abgeschieden. Rußrinnen und Elektrofilter werden dadurch nicht mehr benötigt. Durch Feststoffabscheidung nach dem erfindungsgemäßen Verfahren über einen Vorquench ist der Feststoffgehalt im gereinigten Prozesswasser so gering (13,11 kg Ruß pro Tonne Acetylen), dass durch kontinuierliches Ausschleusung eines Teilstromes des gereinigten Prozesswasser bevorzugt entsprechend dem bei der partiellen Oxidation anfallenden Reaktionswasser ein kontinuierlicher Betrieb des Verfahrens ohne weitere, aufwändige und wasserintensive Fest-Flüssig-Abtrenneinrichtung, insbesondere Rußrinnen, möglich ist.

Ein ähnlich niedriger Anteil an Feststoffen im Prozesswasser wird bislang nach dem Stand der Technik, beispielsweise entsprechend US 5,824,834, nur bei einer mageren Betriebsweise, das heißt mit einer Sauerstoffzahl von 0,32 und entsprechend unter Inkaufnahme einer niedrigeren Ausbeute an Acetylen, von lediglich 6 %, möglich.

In den nachfolgenden Tabelle 1 ist die Abreicherung durch den Reinigungsschritt der Abtrennung unerwünschter flüssiger und gasförmiger Nebenprodukte in einen Kühlturm in Prozenten angegeben.

**Tabelle 1:**

| | Kühlturm | Abreicherung |
|---|---|---|
| | kg/t Acetylen | % |
| CO | 1.20E-04 | 99.9820 |
| Methan | 3.53E-05 | 99.9750 |
| Ethan | 5.39E-06 | 99.9726 |
| Ethylen | 3.55E-05 | 99.9002 |
| Acetylen | 6.67E-02 | 98.9253 |
| Propen | 1.99E-06 | 99.9172 |
| Propadien | 3.78E-06 | 99.9172 |
| Propin | 1.37E-03 | 98.6727 |
| Butenin | 3.90E-04 | 99.1785 |
| Butadiin | 3.62E-02 | 95.9707 |
| Benzol | 3.36E-03 | 97.9296 |
| Naphthalin | 1.01E-04 | 99.3007 |

Aufgrund der hohen Abreicherungsrate kann der Kühlturm durch einen geschlossenen Wärmetauscher ersetzt werden, ohne dass es im Prozess zu nicht tolerablen Aufpegelungen von polymerisierbaren Komponenten, insbesondere von höheren Acetylenen, kommt.

| | Nebenkomponenten im Prozesswasser | |
|---|---|---|
| | geschlossener Wasserquench ohne flash [Gew.ppm] | geschlossener Wasserquench mit flash [Gew.ppm] |
| CO | 1.846 | 0.001 |
| Methan | 0.430 | 0.000 |
| Ethan | 0.061 | 0.000 |
| Ethylen | 0.146 | 0.000 |
| Acetylen | 30.537 | 0.333 |
| Propen | 0.010 | 0.000 |
| Propadien | 0.018 | 0.000 |
| Propin | 0.514 | 0.007 |
| Butenin | 0.233 | 0.002 |
| Butadiin | 4.606 | 0.182 |
| Benzol | 0.018 | 0.017 |
| Naphthalin | 0.071 | 0.001 |

## Patentansprüche

1. Kontinuierliches Verfahren zur Herstellung von Acetylen und Synthesegas durch partielle Oxidation von Kohlenwasserstoffen mit Sauerstoff, wobei ein erster Einsatzstrom (1), enthaltend einen oder mehrere Kohlenwasserstoffe, und ein zweiter Sauerstoff enthaltender Einsatzstrom (2)
- getrennt voneinander, in Vorheizern (V1, V2), aufgeheizt, in einem Verhältnis der Massenströme des zweiten Einsatzstromes (2) zum ersten Einsatzstrom (1) entsprechend einer Sauerstoffzahl von kleiner oder gleich 0,31 in einer Mischeinrichtung gemischt werden, wobei man unter Sauerstoffzahl das Verhältnis aus der tatsächlich im zweiten Einsatzstrom (2) vorhandenen Sauerstoffmenge zur stöchiometrisch notwendigen Sauerstoffmenge versteht, die für die vollständige Verbrennung des einen oder der mehreren, im ersten Einsatzstrom (1) enthaltenen Kohlenwasserstoffe erforderlich ist,
- über einen Brennerblock (BR) einem Feuerraum (FR) zugeführt werden, worin die partielle Oxidation der Kohlenwasserstoffe stattfindet,
- unter Erhalt eines ersten Spaltgasstromes I_{g}
**dadurch gekennzeichnet, dass**
- der erste Spaltgasstrom I_{g} in einem Vorquench (H) durch Eindüsen eines wässrigen Quenchmediums auf eine Temperatur im Bereich von 100 bis 1.000 °C, unter Erhalt eines zweiten Spaltgasstromes II_{g} abgekühlt wird,
- aus dem zweiten Spaltgasstromes II_{g} in einem Feststoff-Gas-Trennapparat (A) 50 bis 90 % der darin enthaltenen Feststoffe abgetrennt werden, unter Erhalt eines Feststoffstromes I_{f} sowie eines dritten Spaltgasstromes III_{g},
- der dritte Spaltgasstrom III_{g} in einem Totalquench (B) durch Eindüsen von Wasser auf 80 bis 90 °C abgekühlt wird, unter Erhalt eines vierten Spaltgasstromes IV_{g} sowie eines ersten Prozesswasserstromes I_{liq},
- der vierte Spaltgasstrom IV_{g} in einem oder mehreren Waschapparaten (C, D) einer Feinabtrennung von Feststoff unterworfen wird, unter Erhalt eines oder mehrerer Prozesswasserströme II_{liq}, III_{liq} sowie eines Produktgasstromes VI_{g},
- die Prozesswasserströme I_{liq}, II_{liq}, III_{liq} zu einem vereinigten Prozesswasserstrom IV_{liq} zusammengeführt werden,
- der vereinigte Prozesswasserstrom IV_{liq} teilweise, als Strom V_{liq}, in den Totalquench (B) zurückgeführt und im Übrigen, als Strom VI_{liq}, einer Reinigung durch Teilverdampfung in einem einstufigen Entspannungsbehälter (E) unterworfen wird, wobei der Strom VI_{liq} zu einem Anteil von 0,01 bis 10 Gew.-%, bezogen auf das Gesamtgewicht desselben, verdampft wird, unter Erhalt eines gereinigten Prozesswasserstromes VII_{liq}, der über eine Rückkühleinrichtung (F) abgekühlt, teilweise, als Strom VIII_{liq} in einen oder mehrere des einen oder der mehreren Waschapparate (C, D) rezykliert, und im Übrigen, als Strom IX_{liq}, ausgeschleust und dem behandlungsbedürftigen Abwasser zugeführt wird.

2. Kontinuierliches Verfahren nach Anspruch 1, **dadurch gekennzeichnet, dass** der Strom IX_{liq} in einem Mengenstrom ausgeschleust wird, der dem bei der partiellen Oxidation der Kohlenwasserstoffe mit Sauerstoff anfallenden Reaktionswasser entspricht.

3. Kontinuierliches Verfahren nach einem der Ansprüche 1 oder 2, **dadurch gekennzeichnet, dass** aus dem zweiten Spaltgasstrom II_{g} im Feststoff-Gas-Trennapparat (A) 60 bis 80 % der darin enthaltenen Feststoffe abgetrennt werden.

4. Kontinuierliches Verfahren nach einem der Ansprüche 1 bis 3, **dadurch gekennzeichnet, dass** der Feststoffstrom I_{f} überwiegend Teere, Ruße und Kokse enthält.

5. Kontinuierliches Verfahren nach einem der Ansprüche 1 bis 4, **dadurch gekennzeichnet, dass** der Feststoff-Gas-Trennapparat (A) ein Zyklonabscheider ist.

6. Kontinuierliches Verfahren nach einem der Ansprüche 1 bis 5, **dadurch gekennzeichnet, dass** der erste Spaltgasstrom I_{g} im Vorquench (H) auf eine Temperatur im Bereich von 200 bis 650 °C abgekühlt wird.

7. Kontinuierliches Verfahren nach Anspruch 6, **dadurch gekennzeichnet, dass** der erste Spaltgasstrom I_{g} im Vorquench (H) auf eine Temperatur im Bereich von 250 bis 400 °C abgekühlt wird.

8. Kontinuierliches Verfahren nach einem der Ansprüche 1 bis 7, **dadurch gekennzeichnet, dass** der Vorquench (H) ein Wasserquench ist.

9. Kontinuierliches Verfahren nach Anspruch 8, **dadurch gekennzeichnet, dass** der Vorquench (H) einen Wärmetauscher zur Auskopplung von Reaktionswärme in Form von Hochdruckdampf umfasst.

10. Kontinuierliches Verfahren nach einem der Ansprüche 1 bis 9, **dadurch gekennzeichnet, dass** als erster Waschapparat (C) ein Venturiwäscher und als zweiter Waschapparat (D) eine Quenchkolonne eingesetzt wird.

11. Kontinuierliches Verfahren nach einem der Ansprüche 1 bis 10, **dadurch gekennzeichnet, dass** der gereinigte Prozesswasserteilstrom VIII_{liq} dem zweiten Waschapparat (D) zugeführt wird.

12. Kontinuierliches Verfahren nach einem der Ansprüche 1 bis 11, **dadurch gekennzeichnet, dass** die Rückkühleinrichtung (F) eine geschlossene Rückkühleinrichtung ist.

13. Kontinuierliches Verfahren nach einem der Ansprüche 1 bis 12, **dadurch gekennzeichnet, dass** die Rückkühleinrichtung (F) ein offener Kühlturm ist.

## Claims

1. A continuous process for preparing acetylene and synthesis gas by partial oxidation of hydrocarbons with oxygen, in which a first input stream (1) comprising one or more hydrocarbons and a second input stream (2) comprising oxygen
- are separately heated in preheaters (V1, V2), mixed in a mixing unit in a ratio of the mass flow of the second input stream (2) to the first input stream (1) corresponding to an oxygen ratio of less than or equal to 0.31, the oxygen ratio being understood to mean the ratio of the amount of oxygen actually present in the second input stream (2) to the amount of oxygen which is needed stoichiometrically and is required for the full combustion of the one or more hydrocarbons present in the first input stream (1),
- supplied via a burner block (BR) to a combustion chamber (FR) in which the partial oxidation of the hydrocarbons takes place,
- to obtain a first cracking gas stream I_{g}, wherein
- the first cracking gas stream I_{g} is cooled in a prequench (H) by injection of an aqueous quench medium to a temperature in the range from 100 to 1000°C to obtain a second cracking gas stream II_{g},
- in a solid-gas separation apparatus (A), 50 to 90% of the solids present in the second cracking gas stream II_{g} are removed therefrom to obtain a solids stream I_{f} and a third cracking gas stream III_{g},
- the third cracking gas stream III_{g} is cooled in a total quench (B) by injection of water to 80 to 90°C to obtain a fourth cracking gas stream IV_{g} and a first process water stream I_{liq},
- the fourth cracking gas stream IV_{g} is subjected in one or more scrubbing apparatuses (C, D) to a fine removal of solids to obtain one or more process water streams II_{liq}, III_{liq} and a product gas stream VI_{g},
- the process water streams I_{liq}, II_{liq}, III_{liq} are combined to give a combined process water stream IV_{liq},
- the combined process water stream IV_{liq} is recycled partly as stream V_{liq} into the total quench (B) and the rest is subjected as stream VI_{liq} to cleaning by partial vaporization in a one-stage flash vessel (E), stream VI_{liq} being vaporized in a proportion of 0.01 to 10% by weight, based on the total weight thereof, to obtain a cleaned process water stream VII_{liq} which is cooled by means of a re-cooling device (F), partly recycled as stream VIII_{liq} into one or more of the one or more scrubbing apparatuses (C, D), and the rest is discharged as stream IX_{liq}, and supplied to the wastewater in need of treatment.

2. The continuous process according to claim 1, wherein stream IX_{liq} is discharged at a flow rate corresponding to the water of reaction obtained in the partial oxidation of the hydrocarbons with oxygen.

3. The continuous process according to either of claims 1 and 2, wherein 60 to 80% of the solids present in the second cracking gas stream II_{g} is separated therefrom in the solid-gas separation apparatus (A).

4. The continuous process according to any of claims 1 to 3, wherein the solids stream I_{f} comprises predominantly tars, soots and cokes.

5. The continuous process according to any of claims 1 to 4, wherein the solid-gas separation apparatus (A) is a cyclone separator.

6. The continuous process according to any of claims 1 to 5, wherein the first cracking gas stream I_{g} is cooled in the prequench (H) to a temperature in the range from 200 to 650°C.

7. The continuous process according to claim 6, wherein the first cracking gas stream I_{g} is cooled in the prequench (H) to a temperature in the range from 250 to 400°C.

8. The continuous process according to any of claims 1 to 7, wherein the prequench (H) is a water quench.

9. The continuous process according to claim 8, wherein the prequench (H) comprises a heat exchanger for extracting heat of reaction in the form of high-pressure steam.

10. The continuous process according to any of claims 1 to 9, wherein the first scrubbing apparatus (C) used is a Venturi scrubber and the second scrubbing apparatus (D) a quench column.

11. The continuous process according to any of claims 1 to 10, wherein the cleaned process water substream VIII_{liq} is supplied to the second scrubbing apparatus (D).

12. The continuous process according to any of claims 1 to 11, wherein the re-cooling device (F) is a closed re-cooling device.

13. The continuous process according to any of claims 1 to 12, wherein the re-cooling device (F) is an open cooling tower.

## Revendications

1. Procédé continu de fabrication d'acétylène et d'un gaz de synthèse par oxydation partielle d'hydrocarbures avec de l'oxygène, selon lequel un premier courant d'entrée (1), contenant un ou plusieurs hydrocarbures, et un deuxième courant d'entrée (2) contenant de l'oxygène
- sont chauffés séparément l'un de l'autre dans des préchauffeurs (V1, V2), mélangés dans un dispositif de mélange en un rapport entre les courants massiques du deuxième courant d'entrée (2) et du premier courant d'entrée (1) correspondant à un indice d'oxygène inférieur ou égal à 0,31, l'indice d'oxygène se rapportant au rapport entre la quantité d'oxygène réellement présente dans le deuxième courant d'entrée (2) et la quantité d'oxygène stoechiométriquement nécessaire pour la combustion totale du ou des hydrocarbures contenus dans le premier courant d'entrée (1),
- introduits par le biais d'un bloc brûleur (BR) dans une chambre de combustion (FR), dans laquelle l'oxydation partielle des hydrocarbures a lieu,
- pour obtenir un premier courant de gaz de clivage I_{g},
**caractérisé en ce que**
- le premier courant de gaz de clivage I_{g} est refroidi dans une pré-trempe (H) par injection d'un milieu de trempe aqueux à une température dans la plage allant de 100 à 1 000 °C, pour obtenir un deuxième courant de gaz de clivage II_{g},
- 50 à 90 % des solides contenus sont séparés du deuxième courant de gaz de clivage II_{g} dans un appareil de séparation solide-gaz (A), pour obtenir un courant de solides I_{f} et un troisième courant de gaz de clivage III_{g},
- le troisième courant de gaz de clivage III_{g} est refroidi dans une trempe totale (B) par injection d'eau à 80 à 90 °C, pour obtenir un quatrième courant de gaz de clivage IV_{g}, ainsi qu'un premier courant d'eau de procédé I_{liq},
- le quatrième courant de gaz de clivage IV_{g} est soumis dans un ou plusieurs appareils de lavage (C, D) à une séparation fine de solides, pour obtenir un ou plusieurs courants d'eau de procédé II_{liq}, III_{liq}, ainsi qu'un courant de gaz produit VI_{g},
- les courants d'eau de procédé I_{liq}, II_{liq} et III_{liq} sont rassemblés en un courant d'eau de procédé réuni IV_{liq},
- le courant d'eau de procédé réuni IV_{liq} est recyclé en partie, en tant que courant V_{liq}, dans la trempe totale (B), et soumis pour le reste, en tant que courant VI_{liq}, à une purification par évaporation partielle dans un contenant de détente à une étape (E), le courant VI_{liq} étant évaporé en une proportion de 0,01 à 10 % en poids, par rapport à son poids total, pour obtenir un courant d'eau de procédé purifié VII_{liq}, qui est refroidi par un dispositif de refroidissement à retour (F), recyclé en partie, en tant que courant VIII_{liq}, dans un ou plusieurs du ou des appareils de lavage (C, D), et pour le reste, en tant que courant IX_{liq}, déchargé et introduit dans l'eau résiduaire nécessitant un traitement.

2. Procédé continu selon la revendication 1, **caractérisé en ce que** le courant IX_{liq} est déchargé en une proportion qui correspond à l'eau de réaction formée lors de l'oxydation partielle des hydrocarbures avec de l'oxygène.

3. Procédé continu selon l'une quelconque des revendications 1 ou 2, **caractérisé en ce que** 60 à 80 % des solides contenus sont séparés du deuxième courant de gaz de clivage II_{g} dans l'appareil de séparation solide-gaz (A).

4. Procédé continu selon l'une quelconque des revendications 1 à 3, **caractérisé en ce que** le courant de solides I_{f} contient principalement des goudrons, des noirs de carbone et des cokes.

5. Procédé continu selon l'une quelconque des revendications 1 à 4, **caractérisé en ce que** l'appareil de séparation solide-gaz (A) est un séparateur à cyclone.

6. Procédé continu selon l'une quelconque des revendications 1 à 5, **caractérisé en ce que** le premier courant de gaz de clivage I_{g} est refroidi dans la pré-trempe (H) à une température dans la plage allant de 200 à 650 °C.

7. Procédé continu selon la revendication 6, **caractérisé en ce que** le premier courant de gaz de clivage I_{g} est refroidi dans la pré-trempe (H) à une température dans la plage allant de 250 à 400 °C.

8. Procédé continu selon l'une quelconque des revendications 1 à 7, **caractérisé en ce que** la pré-trempe (H) est une trempe à l'eau.

9. Procédé continu selon la revendication 8, **caractérisé en ce que** la pré-trempe (H) comprend un échangeur de chaleur pour le découplage de la chaleur de réaction sous la forme de vapeur haute pression.

10. Procédé continu selon l'une quelconque des revendications 1 à 9, **caractérisé en ce qu'**un laveur Venturi est utilisé en tant que premier appareil de lavage (C), et une colonne de trempe en tant que deuxième appareil de lavage (D).

11. Procédé continu selon l'une quelconque des revendications 1 à 10, **caractérisé en ce que** le courant partiel d'eau de procédé purifié VIII_{liq} est introduit dans le deuxième appareil de lavage (D).

12. Procédé continu selon l'une quelconque des revendications 1 à 11, **caractérisé en ce que** le dispositif de refroidissement à retour (F) est un dispositif de refroidissement à retour fermé.

13. Procédé continu selon l'une quelconque des revendications 1 à 12, **caractérisé en ce que** le dispositif de refroidissement à retour (F) est une tour de refroidissement ouverte.
